# EUROPEAN PATENT APPLICATION

(11) **EP 1 202 063 A1**
(43) Date of publication of application: **02.05.2002**
(21) Application number: 99973928.7
(22) Date of filing: 25.06.1999
(51) Int. Cl.: G01N 33/552, G01N 33/551, G01N 33/543

(54) **IMMUNOASSAY SUPPORTS AND IMMUNOASSSAY SOLID PHASES WITH THE USE THEREOF**

(71) Applicant: KYOWA MEDEX CO., LTD., Tokyo 104-0042 (JP)
(72) Inventor: KUMAZAWA, Toshiaki, Hachioji-shi, Tokyo 192-0031 (JP); TAGAMI, Hiroaki, Hachioji-shi, Tokyo 192-0031 (JP); KIYA, Yoshiyasu, Hachioji-shi, Tokyo 192-0031 (JP); YOKOHAMA, Hiroaki, Kyowa Medex Co., Ltd., Sunto-gun, Shizuoka 411-0932 (JP); MORI, Hideharu, Kyowa Medex Co., Ltd., Sunto-gun, Shizuoka 411-0932 (JP); MATSUMORI, Shigeru, Kyowa Medex Co., Ltd., Sunto-gun, Shizuoka 411-0932 (JP)
(74) Representative: VOSSIUS & PARTNER
(86) International application number: JP9903427
(87) International publication number: WO0101145

(57) **Abstract**

Disclosed is a novel carrier for immunoassay which is applicable irrespective of the composition of glass fibers, and which can make the assay sensitivity higher than that in the cases where a known carrier for immunoassay using glass fibers is used. The carrier for immunoassay of the present invention comprises, at least on the surface thereof, a silicon compound of the following Formula [I] and/or [II]. 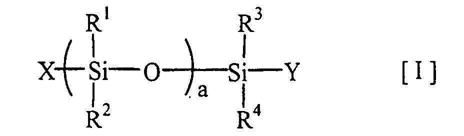 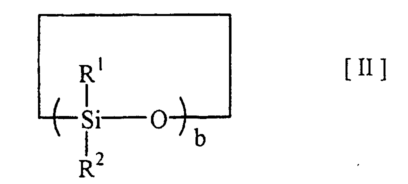 (In Formulae [I] and [II], R¹ to R⁴, X and Y independently represent hydrogen or substituted or non-substituted organic group, a is an integer of 0 to 5000, and b is an integer of 3 to 20).

## Description

### Technical Field

The present invention relates to a carrier for immunoassay and solid phase for immunoassay containing the same.

### Background Art

Heretofore, immunoassay for measuring antigens and antibodies, especially those employing solid carriers, are widely used. As the solid carriers employed in immunoassay, polystyrene beads, wells in microplates, etc. are usually used.

However, in the conventional immunoassay in which an immunoreactive substance such as an antigen or antibody is immobilized on polystyrene beads, wells in microplates or the like, it takes 30 to 180 minutes for the immunological reaction, and further, it takes 30 to 180 minutes for the reaction with the labeled antibody, and they are considerably time-consuming. Further, a large amount of washing solution, that is, 1000 to 10,000 µl of washing solution is necessary for the B/F separation.

In systems for measuring antibodies, when a specific antibody is used as the labeled substance, a measuring system in which the influence by non-specific adsorption is small can be attained. However, when an anti-human IgG antibody which can be commonly used for various immunoassay is used as the labeled substance in the system for measuring antibodies against various infectious diseases, non-specific adsorption of the IgG originated from sera is large.

It is known that by using glass fibers having a specific composition as the carrier, the time needed for the immunoassay can be made much shorter than that in the conventional immunoassay, and the amounts of the washing solution and the samples can be made smaller than that in the conventional immunoassay (Japanese Laid-open Patent Application (Kokai) No. 10-104237). However, in this method, the composition of the glass fibers is restricted. Further, needless to say, it is advantageous if a measurement sensitivity higher than that in this known method is attained.

### Disclosure of the Invention

Accordingly, an object of the present invention is to provide a novel carrier for immunoassay which is applicable irrespective of the composition of the glass fibers, and which can make the assay sensitivity higher than that in the cases where a known carrier for immunoassay employing glass fibers is used.

Another object of the present invention is to provide a carrier for immunoassay, which can effectively prevent the non-specific adsorption of, for example, IgG originated from sera.

The present inventors intensively studied to discover that assay sensitivity is significantly improved by using a carrier for immunoassay which comprises, at least on surface thereof, a particular silicon compound, and the desired assay sensitivity may be attained even if the amount of the expensive immunoreactive substance immobilized on the carrier is largely decreased, thereby completing the present invention.

That is, the present invention provides a carrier for immunoassay which comprises, at least on surface thereof, a silicon compound of the following Formula [I] and/or [II]. 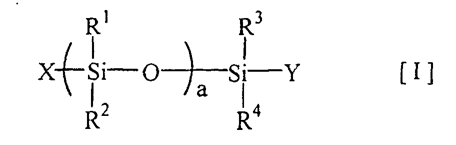 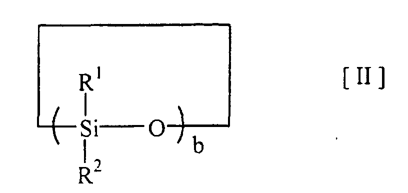 (In Formulae [I] and [II], R¹ to R⁴, X and Y independently represent hydrogen or substituted or non-substituted organic group, a is an integer of 0 to 5000, and b is an integer of 3 to 20).

The present inventors also discovered as a result of intensive study that non-specific adsorption can be significantly reduced by forming at least the surface of the carrier for immunoassay with a hydrophobic substance and by using an amphipathic substance as a blocking agent, thereby completing the present invention.

That is, the present invention provides a solid phase for immunoassay obtainable by binding an immunoreactive substance to a carrier for immunoassay which comprises, at least on surface thereof, a hydrophobic material, and blocking the resulting carrier by coating exposed area of said hydrophobic material with an amphipathic substance.

By the present invention, a novel carrier for immunoassay which comprises, at least on surface thereof, a particular silicon compound, as well as a method for immobilizing a ligand thereon, was provided. According to the present invention, by constituting the surface of the carrier with a particular silicon compound, the assay sensitivity is significantly improved compared with those in the immunoassay using the conventional carriers, so that the amount of the ligand immobilized on the carrier which is necessary to attain the same level of sensitivity may be significantly decreased. Further, since the surface treated with the silicon compound very well adsorbs surfactants, especially nonionic surfactants such as polyoxyethylenesorbitan alkyl esters, blocking may be carried out using such a surfactant. For blocking of the carriers for immunoassay, proteins such as BSA or gelatin are conventionally used, however, a surfactant is more advantageous in view of the cost and storage of the reagent than using these proteins originated from living organisms. Further, by using the carrier according to the present invention, immunoassays can be carried out in shorter time, and the washing solution and samples can be decreased compared with the conventional immunoassays using the wells in microplates or beads as the carriers.

Further, according to the present invention, a novel solid phase for immunoassay which comprises, on surface thereof, a hydrophobic material and the surface of which is blocked with an amphipathic substance was provided. With this solid phase for immunoassay, non-specific adsorption can be significantly reduced when compared with the conventional solid phases for immunoassay, so that the assay sensitivity is high.

### Best Mode for Carrying Out the Invention

The silicon compound used in the present invention has the structure represented by the above-described Formula [I] or [II]. In Formulae [I] and [II], R¹ to R⁴, X and Y independently represent hydrogen or substituted or non-substituted organic group, a is an integer of 0 to 5000, and b is an integer of 3 to 20. Preferably, R¹ to R⁴ independently represent hydrogen, C₁-C₆ alkyl, C₁-C₆ alkoxyl or phenyl; and X and Y independently represent hydrogen, silanol, C₁-C₆ alkoxyl or amino. Examples of the especially preferred silicon compounds within this category include dialkylpolysiloxanes (especially dimethylpolysiloxanes) and modified dialkylpolysiloxane (especially modified dimethylpolysiloxane) in which one or both ends of dialkylpolysiloxanes (especially dimethylpolysiloxanes) is substituted with silanol, C₁-C₆ alkoxyl or amino. Also preferred are the hydrophobic silanes represented by the above-described Formula [I] wherein a is 0; X, Y, R³ and R⁴ independently represent hydrogen or an organic group, and at least one of them is a hydrophobic organic group. Among these, the hydrophobic silanes wherein the silicon compound is a hydrophobic silane represented by Formula [I] wherein X and Y independently represent hydrogen, C₁-C₆ alkyl, or C₁-C₆ alkoxyl; R³ is C₂-C₃₀ alkyl, C₂-C₃₀ alkenyl, C₂-C₃₀ alkoxyl, phenyl or substituted phenyl; R⁴ is hydrogen, C₁-C₃₀ alkyl or C₁-C₃₀ alkoxyl, are especially preferred. Examples of the especially preferred silicon compounds within this category include alkyltrialkoxysilanes, in particular, octadecyltriethoxysilane, vinyltriethoxysilane and phenyltriethoxysilane. The silicon compounds preferably have molecular weights of about 5000 to 10,000.

The carrier for immunoassay according to the present invention comprises, at least on the surface thereof, the above-described silicon compound. Although the entire carrier may be made of the above-described silicon compound, coating a substrate with the silicon compound is simple and preferred. In this case, the substrate is not restricted at all, and carriers used in the conventional immunoassay, for example, inner walls of various reaction vessels such as wells of microtiter plates, and carriers in the form of beads or membranes, may be employed. The material of the substrate is not restricted, and hydrophobic polymers such as polystyrene, which are widely used in this field, as well as inorganic materials, may be employed. Glass, quartz and ceramics are preferred, and glass is especially preferred as the material of the substrate. To increase the area of the surface on which the immunological reactions occur, the substrate may preferably be porous such as in the form of a membrane. Thus, as the substrate, those in the form of a membrane made of glass fibers, quartz fibers or ceramics fibers, especially made of glass fibers, are especially preferred.

The diameter of the glass fibers used as the substrate is not restricted. Usually, a diameter of 0.1 to 100 µm is suitable, and 0.5 to 2.0 µm is preferred. The diameter of the particles to be retained may preferably be about 0.1 to 10 µm. Although the glass fibers may be in any form, those in the form of a membrane are easy to handle and preferred. In this case, the basis weight of the membrane is not restricted, and about 90 to 130 g/m² is suitable. The thickness of the membrane is not restricted, and about 0.3 mm to 0.6 mm is suitable. The composition of the glass constituting the glass fibers is not restricted. Some glass fiber membranes are commercially available, and the commercially available glass fiber membranes may preferably be employed as the substrate of the carrier for immunoassay according to the present invention.

The treatment with the silicon compound may be carried out by immersing the substrate such as glass fibers in a solution of the silicon compound, by spraying the silicon compound solution, or by applying the silicon compound solution by using a spinner or the like. The solvent of the silicon compound solution may be any solvent which can dissolve the silicon compound, and water and water-soluble organic solvents such as C₁-C₄ lower aliphatic alcohols are preferred. The concentration of the silicon compound in the solution may usually be about 0.01 to 90% by weight, preferably about 0.1 to 10% by weight. The treatment may be carried out at any temperature at which the silicon compound solution is kept in the form of liquid, and treatment at room temperature is the easiest to carry out.

After the treatment, the substrate is dried. The drying is satisfied if the removal of the aqueous organic solvent is attained. The drying may usually be carried out at room temperature to 200°C for about 5 minutes to 120 minutes. The drying may be carried out in a dryer or in a room.

After the drying, an immunoreactive substance is immobilized. Since the above-described silicon compounds are water-repelling, in order to accelerate immobilization of the immunoreactive substance, it is preferred to treat the carrier with a water-soluble organic solvent and with water successively, prior to immobilization. Examples of the water-soluble organic solvent includes C₁-C₄ lower aliphatic alcohols (especially ethanol and propanol), dimethylformamide and dimethylsulfoxide, as well as aqueous solutions of these solvents. This treatment may be carried out at any temperature at which the aqueous organic solvent and water are kept in the form of liquid, and the treatment at room temperature is simplest.

The immunoreactive substance (hereinafter also referred to as "ligand") to be immobilized to a carrier may be any substances which can undergo antigen-antibody reaction. Thus, this term includes not only antigens and antibodies, but also includes fragments thereof, e.g., haptens and Fab fragment and F(ab')₂ fragment of antibodies. The immobilization may be conducted by contacting the carrier with a solution of the ligand in a buffer. In this case, the immobilization reaction may be carried out, as in the conventional method, at room temperature for about 15 minutes to 2 hours, or at 4°C overnight. The concentration of the ligand solution used in the immobilization may appropriately be selected depending on the type of the ligand and the types and concentrations of the substances in the sample, and usually, about 0.5 to 200 µg/ml.

Blocking is then carried out. Blocking may be carried out in a conventional way. Blocking may be carried out using a protein such as BSA, casein or gelatin as in the conventional methods. Alternatively, as the blocking agent, amphipathic substances such as surfactants may also be employed. The amphipathic substance is preferred because non-specific adsorption may be significantly reduced. Further, a surfactant is more advantageous than the proteins originated from living organisms in view of the cost and storage of the reagent. Depending on the type of the silicon compound, blocking may not be well accomplished if a hydrophilic protein such as BSA is used as the blocking agent. In such a case, an amphipathic substance or a hydrophobic protein (e.g., Block Ace commercially available from SNOW BRAND MILK PRODUCTS CO., LTD.) is used.

Examples of the amphipathic substances include nonionic, anionic, cationic and amphoteric surfactants. Examples of the nonionic surfactants include linear alkylpolyoxyethylene ethers, sorbitan-alkylpolyoxyethylene ethers, and alkylphenylpolyoxyethylene ethers. Examples of the anionic surfactants include sodium oleate, sodium cholate, sodium dodecylsulfate, dipalmitoylphosphatidic acid, dipalmitoylphosphatidylserine, Persoft EL (produced by NIPPON OIL & FATS CO., LTD). Examples of the cationic surfactants include Cation AB (produced by NIPPON OIL & FATS CO., LTD). Examples of the amphoteric surfactants include dimyristoylphosphatidylcholine (DMPC), dipalmitoylphosphatidylcholine (DPPC), distearoylphosphatidylcholine (DSPC) and egg yolk phosphatidylcholine.

Among these amphipathic substances, preferred are nonionic surfactants having polyoxyethylene chains at hydrophilic groups, such as polyoxyethylenesorbitan monolaurate (trademark Tween 20), polyoxyethylenesorbitan monopalmitate (trademark Tween 40) and polyoxyethylenesorbitan monostearate (trademark Tween 60). Blocking may be completed by adding to the carrier a solution of such a surfactant preferably having a concentration of 0.1 to 2% by weight, in an amount of 50 to 150 µl in case of; for example, using a glass filter having a diameter of 8 mm, and leaving the resultant mixture to stand at room temperature for about several minutes to 24 hours. The resultant mixture may be left to stand under reduced pressure.

In cases where the amphipathic substance is used as the blocking agent, the non-specific adsorption may be significantly reduced even when the surface of the carrier is made of a hydrophobic material other than the above-described silicon compound, and the immunoassay may be carried out with a higher sensitivity than in the conventional immunoassay. Therefore, the solid phase for immunoassay obtainable by binding an immunoreactive substance to a carrier for immunoassay which comprises, at least on the surface thereof, a hydrophobic material, and blocking the resulting carrier by coating the exposed area of the hydrophobic material with an amphipathic substance is also within the scope of the present invention. In this case, the hydrophobic material includes hydrophobic polymers such as polystyrene and polyolefins, other than the above-described silicon compounds.

The carrier for immunoassay according to the present invention may be employed in any immunoassay using solid carriers, and the immunoassay *per se* using the carrier of the present invention may be carried out basically in the same way as in the conventional immunoassay. Thus, on the carrier for immunoassay of the present invention, the ligand is immobilized and then the resultant solid phase is subjected to the blocking as described above. The solid phase obtained by immobilizing the ligand on the carrier for immunoassay of the present invention may be used in, for example, sandwich assays as the conventional solid phases.

When used in the sandwich assay, the carrier of the present invention on which the ligand is immobilized reacts with a sample as in the conventional method after conducting the blocking as mentioned above. After washing the carrier, a second antibody is subjected to reaction. After washing, the second antibody remaining on the solid phase is measured. The measurement of the second antibody may be carried out by preliminarily labeling the second antibody with a fluorescent pigment, biotin, radioactive substance or the like, and detecting the label. It should be noted that the carrier for immunoassay of the present invention may be applied not only to sandwich assay, but also to any immunoassay using an immobilized ligand.

As will be specifically described in the Examples below, by treating glass fibers with an aqueous organic solvent, the assay sensitivity is significantly increased, so that the amount of the ligand immobilized on the carrier which is necessary to attain the same level of sensitivity may be significantly decreased (e.g., to 1/50 to 1/100). Since ligands are expensive, it is a great advantage to decrease the amount of the ligand.

The reason why the sensitivity of the immunoassay is improved by treating the carrier with the silicon compound has not been clarified. Presumably, this is because that the amount of the ligand which can be immobilized is increased, since the affinity between the silicon compound and the immunoreactive substance is high.

By using the amphipathic substance as the blocking agent, the non-specific adsorption of IgG or the like may be reduced to 1/10 or less compared with the solid phase blocked with BSA or the like.

### Examples

The present invention will now be described more specifically referring to examples. However, the present invention is not restricted to the Examples.

### Example 1

### (1) Treatment of Glass Fiber Carrier

A commercially available glass fiber membrane (glass fiber membrane GA100 produced by ADVANTEC, diameter of glass fibers: 0.8 µm to 1.5 µm, basis weight: 110 g/m², thickness: 0.45 mm, size: 300 mm x 200 mm) was immersed in aqueous dimethylpolysiloxane ("Siliconized L-25" produced by SHIN-ETSU-CHEMICAL CO., LTD.) solution having a concentration of 0.1% by weight for 15 seconds. After washing the membrane with 500 ml of pure water 5 times, the membrane was dried in an oven kept at 120°C for 1 hour. The membrane was cut into disks with a diameter of 6 mm, and the obtained disks were used as the carriers in the reaction vessels of an automatic immunoassay apparatus (ID-1000 produced by KYOWA MEDEX CO., LTD).

### (2) Immobilization of Core Antigen of Hepatitis C Virus (HCV)

As a pretreatment before adding the ligand dropwise, 20 µl of propanol was added dropwise, and then 30 µl of pure water was added dropwise. As the ligand, HCV core peptide consisting of 40 amino acids was dissolved in 0.01M PBS (pH 7) to a concentration of 1 µg/ml or 100 µg/ml, and 60 µl of each of the prepared solutions was added dropwise, and was allowed to react at room temperature for 30 minutes. Thereafter, 80 µl of 10% Tween 20 (trademark) in 0.01 M PBS (pH 7) was added dropwise, and was allowed to react at room temperature for 30 minutes, thereby carrying out blocking.

### (3) Immunoassay

Each of the serum samples which have been assayed to be positive or negative for anti-HCV antibody by another assay, was diluted by 50-fold, and 50 µl aliquot thereof was subjected to the reaction. The reaction was carried out at room temperature for 2 minutes, and the solid phase was then washed with 50 µl of washing solution. Then, 50 µl of dilution of a peroxidase-labeled antibody was added dropwise, and the resultant mixture was allowed to react at room temperature for 2 minutes, followed by addition of 80 µl of a washing solution dropwise twice, thereby carrying out B/F separation. Then, 30 µl of tetramethylbenzidine (TMB) as the substrate of peroxidase was added dropwise. From the above-mentioned automatic immunoassay apparatus, ΔK/s value was obtained. The ΔK/s value is the value measured by ID-1000 produced by KYOWA MEDEX CO., LTD., and is almost completely the same as the absorbance measured in ordinary immunoassay. Therefore, ΔK/s value is hereinafter described as "absorbance". The results are shown in Table 1.

### Comparative Example 1

The same operations as in Example 1 were repeated except that the treatment with dimethylpolysiloxane was not conducted. The results are shown in Table 1 below.

**Table 1**

| Example | Sample | Ligand Concentration (µg/ml) | |
|---|---|---|---|
| | | 1 | 100 |
| Example 1 | positive | 2.135 | 5.348 |
| | negative | 0.125 | 0.185 |
| Comparative Example 1 | positive | 0.213 | 2.083 |
| | negative | 0.201 | 0.245 |

As shown in Table 1, with respect to the positive samples, the absorbances in Example 1 were significantly higher than those in Comparative Example 1, while with respect to the negative samples, the absorbances in Example 1 were rather lower than those in Comparative Example 1. Therefore, the assay sensitivity is significantly higher in Example 1 than in Comparative Example 1. Further, the absorbance for the positive sample in Example 1 wherein the ligand concentration was 1 µg/ml was almost the same as that in Comparative Example 1 wherein the ligand concentration was 100 µg/ml, and therefore, the amount of the ligand in Example 1 needed for obtaining the same level of assay sensitivity as in Comparative Example 1 wherein the ligand concentration was 100 µg/ml was 1/100 of that needed in Comparative Example 1.

### Example 2

### (1) Treatment of Glass Fiber Carrier

A commercially available glass fiber membrane (glass fiber membrane GF/D produced by WHATMAN, basis weight: 120 g/m², thickness: 0.68 mm, size: 150 mm x 150 mm) was immersed in aqueous dimethylpolysiloxane solution having a concentration of 0.1% by weight for 15 seconds, as in Example 1. After washing the membrane with 500 ml of pure water 5 times, the membrane was dried in an oven kept at 120°C for 1 hour. The membrane was cut into disks with a diameter of 6 mm, and the obtained disks were used as the carriers in the reaction vessels of an automatic immunoassay apparatus (ID-1000 produced by KYOWA MEDEX CO., LTD).

### (2) Immobilization of Native Treponema pallidum (TP) Antigen

As a pretreatment before adding the ligand dropwise, 20 µl of propanol was added dropwise, and then 30 µl of pure water was added dropwise. As the ligand, native TP antigen was dissolved in 0.01M PBS (phosphate buffer-containing saline solution) (pH 7) to a concentration of 1 µg/ml or 100 µg/ml, and 60 µl of each of the prepared solutions was added dropwise, and was allowed to react at room temperature for 30 minutes. Thereafter, 80 µl of 10% Tween 20 (trademark) in 0.01 M PBS (pH 7) was added dropwise, and the resultant was allowed to react at room temperature for 30 minutes, thereby carrying out blocking.

### (3) Immunoassay

Immunoassay was carried out in the same way as in Example 1. The results are shown in Table 2 below.

### Comparative Example 2

The same operations as in Example 2 were repeated except that the treatment with dimethylpolysiloxane was not conducted. The results are shown in Table 2 below.

**Table 2**

| Example | Sample | Ligand Concentration (µg/ml) | |
|---|---|---|---|
| | | 1 | 100 |
| Example 2 | positive | 4.265 | 8.215 |
| | negative | 0.118 | 0.271 |
| Comparative Example 2 | positive | 1.135 | 3.998 |
| | negative | 0.233 | 0.267 |

As shown in Table 2, with respect to the positive samples, the absorbances in Example 2 were significantly higher than those in Comparative Example 2, while with respect to the negative samples, the absorbances in Example 2 were rather lower than those in Comparative Example 2. Therefore, the assay sensitivity is significantly higher in Example 2 than in Comparative Example 2. Further, the absorbance for the positive sample in Example 2 wherein the ligand concentration was 1 µg/ml was almost the same as that in Comparative Example 2 wherein the ligand concentration was 100 µg/ml, and therefore, the amount of the ligand in Example 1 needed for obtaining the same level of assay sensitivity in Comparative Example 2 wherein the ligand concentration was 100 µg/ml was 1/100 of that needed in Comparative Example 2.

### Example 3, Comparative Example 3

GF/D sheet (30 x 20 cm, produced by WHATMAN) was immersed in solution containing 0.08% of octadecyltriethoxysilane (produced by SHIN-ETSU CHEMICAL CO., LTD, hereinafter also referred to as "C18") in acetone. The immersed GF/D sheet was taken out with a stainless steel tray, and the sheet was then washed by being immersed in acetone solution, followed by drying under reduced pressure (<5 Torr, 30°C, 24 hours). Further, heat-treatment (100°C, 5 hours) was conducted to obtain GF/D filter modified with octadecyltriethoxysilane.

The obtained octadecyltriethoxysilane-modified GF/D filter was punched with a skin punch to prepare disks with a diameter of 8 mm, and the obtained disks were placed in the reaction cells for ID-1000 (produced by KYOWA MEDEX CO., LTD), respectively. Then, 70 µl of 50% ethanol solution and 150 µl of 10 mM phosphate buffer (pH 7) were successively added. Further, 100 µl aliquot of each of the solutions of the amphiphathic substances shown in Table 3 [1%, in 10 mM phosphate solution (pH 7)] was added as a blocking agent, and dried under reduced pressure (5 Torr or less, room temperature, 24 hours) to prepare cells for assay.

For comparison, GF/D filters not modified with octadecyltriethoxysilane were placed in the reaction cells for ID-1000 (produced by KYOWA MEDEX CO., LTD.), and treated with the amphipathic substances shown in Table 3 in the same way, respectively, to obtain cells for assay (Comparative Example 3).

The cells for assay prepared as described above were tested for the effect for inhibiting non-specific adsorption as follows: As the sample, normal human serum diluted by 2-fold with 10 mM phosphate (pH 7) was used, and the blank value was measured with ID-1000 (KYOWA MEDEX CO., LTD).

Each of the cells for assay prepared by the method described above was set in ID-1000, and 50 µl of blocking solution [10 mM phosphate buffer (pH 7.2)], 50 µl of sample solution, 50 µl of washing solution [10 mM phosphate buffer (pH 7.2), 150 mM sodium chloride, 0.05% Tween 20], 50 µl of label solution [40 mM Tris-HCl buffer (pH 7.0), 0.4% bovine serum albumin, 0.2% bovine serum, 0.2% rabbit serum, POD (peroxidase)-labeled anti-human IgG antibody], 100 µl of the above-mentioned washing solution (twice), and 100 µl of coloring solution [25 mM citrate buffer (pH 5.0), 0.1% Triton X-100, 0.02% hydrogen peroxide, MCDP (10-(N-methylcarbamoyl)-3,7-bis(dimethylamino)phenothiazine)] were successively added.

The change in the reflectance (wavelength: 600 nm) of the filter surface was determined by using an integrating sphere to calculate the ΔK/S value (Kubelka-Munk equation).

The results are shown in Table 3.

**Table 3**

| Surfactant (All are trademarks except for SDS and BSA) | ΔK/S Value | |
|---|---|---|
| | C18-treated GF/D | Non-treated GF/D |
| Tween-60 | 0.174 | 1.925 |
| Brij-56 | 0.233 | 1.446 |
| Triton X-100 | 0.800 | 3.649 |
| Cation AB | 0.704 | 2.661 |
| SDS | 1.074 | 2.965 |
| Persoft EL | 0.464 | 3.309 |
| BSA | * | 2.660 |

| | | |
|---|---|---|
| *): In case of using BSA, measurement cannot be carried out because the hydrophilicity of C18-treated GF/D was insufficient. | | |

In the case that the octadecyltriethoxysilane-modified GF/D filter was blocked with the amphiphatic substance, the ΔK/S value was reduced to about 1/3 to 1/10 times compared with the conventional method wherein GF/D filter is blocked with an amphipathic substance such as BSA.

Similar effect may be obtained by using a nonionic surfactant other than Tween 60, a phospholipid or the like. Further, in cases where 10-fold or 50-fold diluted human serum solution was used, the blank value was smaller when the treatment method of the present invention was employed. In case where a nonionic surfactant such as Tween 60 was added to non-treated GF/D filter, the blank value was about the same as in the case where an aqueous protein was added, and the effect to inhibit non-specific adsorption was small. From this result, it can be seen that it is preferred that the amphipathic substance is added to a carrier which was subjected to a treatment for converting the surface hydrophobic, in order to increase the effect to inhibit non-specific adsorption.

### Example 4, Comparative Example 4 Measurement of Anti-TP (Treponema pallidum) Antibody

The octadecylsilane-modified GF/D filter prepared as in Example 3 was set in the cell for assay of ID-1000, and 70 µl of 50% ethanol solution and then 150 µl of 10 mM phosphate solution (pH 7) were successively added. Then, 100 µl of native-TP antigen (IWAKI & CO., LTD.) solution (5 µg/ml, pH 7, in 10 mM phosphate buffer) was added, and the mixture was allowed to stand at room temperature for 10 minutes. As a blocking solution, 100 µl of Tween 60 solution (1%, pH 7, 10 mM phosphate buffer) which is an amphipathic substance was added, and as a stabilizer for drying, trehalose solution (2.5%, pH 7, 10 mM phosphate buffer) was added. Then the mixture was dried under reduced pressure (5 Torr or less, room temperature, 24 hours) to obtain a cell for assay.

For comparison, a cell in which native-TP antigen was immobilized on GF/D filter by blocking with BSA was prepared. More particularly, GF/D filter in the form of a disk with a diameter of 8 mm was set in a reaction cell, and 50 µl of wetting solution (pH 7, 10 mM phosphate buffer), and then 100 µl of native-TP antigen (produced by IWAKI & CO., LTD.)(5 µg/ml, pH 7, 10 mM phosphate buffer) are successively added. The mixture was allowed to stand at room temperature for 10 minutes, and 50 µl of BSA solution (0.1%, pH 7, 50 mM phosphate buffer, 1% sucrose) is added. The obtained cell was dried under reduced pressure (5 Torr or less, room temperature, 24 hours) to obtain a cell for assay for comparison (Comparative Example 4).

The two types of cells for assay were compared for reactivity using ID-1000. As the samples, serum solutions prepared by diluting a TP-positive serum (produced by TOKIWA CHEMICAL, titer by TPHA method: x 5120) with normal human serum (NHS) (produced by IRVINE) to prescribed titers (x 80 (corresponding to cut off value), x 160, and x 320), and each of which diluted by 50-fold with a sample-diluting solution. Assay was carried out using ID-1000. To the reaction cell, 50 µl of blocking solution, 50 µl of a sample solution (50-fold diluted with serum) , 50 µl of first washing solution, 50 µl of label solution (POD-labeled anti-human IgG antibody, produced by JACKSON), 100 µl x 2 of second washing solution, and then 30 µl of coloring solution (Ultra Blue TMB, produced by INTERGEN) were successively added, and the ΔK/S value was calculated.

The results are shown in Table 4.

**Table 4**

| Sample | Protected with BSA | Treated with C18 |
|---|---|---|
| NHS | 1.099 | 0.086 |
| x 40 | 1.114 | 0.120 |
| x 80 | 1.109 | 0.184 |
| x 160 | 1.128 | 0.294 |
| x 320 | 1.190 | 0.606 |

With respect to the octadecyltriethoxysilane-modified GF/D cell, the ΔK/S value was concentration-dependently increased, and the ΔK/S value of the serum with a titer of x 80 corresponding to the vicinity of the cut off value was significantly higher than the ΔK/S value of normal human serum. In contrast, in case of Comparative Example 4, the blank value for normal human serum was higher, so that detection was difficult.

### Example 5 Measurement of Anti-TP Antibody Using ID-1000

Under the same conditions as in Example 3, the immunoassay was conducted for 50 TP-positive serum samples (titer: x 80 to 20,480) of which titers were determined by using a reagent for detecting anti-TP antibody, SERODIA TP-PA (FUJIREBIO INC.) and for 50 TP-negative serum samples. The obtained ΔK/S values were significantly different between positive group (not less than 0.5) and negative group (not more than 0.2), and thus, all of the tested samples were able to be correctly classified into positive or negative group.

### Example 6, Comparative Example 5 Measurement of Anti-HCV Antibody/Distinguishing Group 1 and Group 2

Cells for reaction were prepared as in Example 2 except that the immobilized antigen was a synthetic peptide antigen ckk-n1 or ckk-n2 (WO97/08198) was used as the immobilized antigen and that the concentration of the antigen solution was 10 µg/ml.

Using ckk-n1-positive and ckk-n2-positive serum samples (20 samples each) which were distinguished by using Immunocheck-HCV Gr Kokusai (produced by INTERNATIONAL REAGENTS CORPORATION), assay was conducted under the same conditions as in Example 4.

In cases where the silane-treated cell was used, with respect to the cells on which the synthetic peptide antigen ckk-n1 was immobilized, the ΔK/S values for the synthetic peptide antigen ckk-n1-positive serum samples were 0.2 or more, while the ΔK/S values for the synthetic peptide antigen ckk-n2-positive serum samples were 0.1 or less, and distinguishing positive and negative samples was attained. Similarly, with respect to the cells in which the synthetic peptide antigen ckk-n2 was immobilized, the ΔK/S values for the synthetic peptide antigen ckk-n1-positive serum samples were 0.1 or less, while the ΔK/S values for the synthetic peptide antigen ckk-n2-positive serum samples were 0.2 or more, so that distinguishing positive and negative samples was attained. In contrast, in cases where the cell for reaction not treated with octadecyltriethoxysilane was used (Comparative Example 5), with respect to the cells immobilized either of the synthetic peptide antigen NS4ckk-n1 or ckk-n2, respectively, the ΔK/S values were 0.5 or more for both the ckk-n1-positive and ckk-n2-positive serum samples, so that distinguishing positive and negative samples could not be attained.

## Claims

1. A carrier for immunoassay which comprises, at least on surface thereof, a silicon compound of the following Formula [I] and/or [II]. 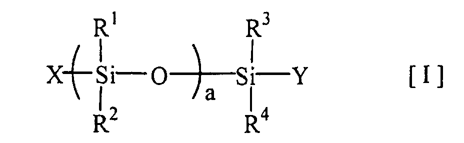 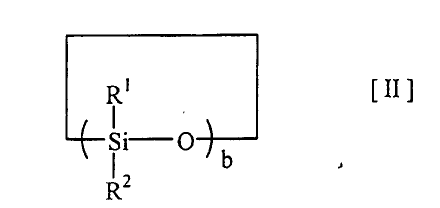 (In Formulae [I] and [II], R¹ to R⁴, X and Y independently represent hydrogen or substituted or non-substituted organic group, a is an integer of 0 to 5000, and b is an integer of 3 to 20).

2. The carrier for immunoassay according to claim 1, wherein in formula [I] or [II], R¹ to R⁴ independently represent hydrogen, C₁-C₆ alkyl, C₁-C₆ alkoxyl or phenyl, and X and Y independently represent hydrogen, silanol, C₁-C₆ alkoxyl or amino.

3. The carrier for immunoassay according to claim 1, wherein said silicon compound is a dialkylpolysiloxane.

4. The carrier for immunoassay according to claim 3, wherein said dialkylpolysiloxane is dimethylpolysiloxane.

5. The carrier for immunoassay according to claim 1, wherein said silicon compound is represented by said Formula [I] wherein a is 0; X, Y, R³ and R⁴ independently represent hydrogen or an organic group, and at least one of them is a hydrophobic organic group.

6. The carrier for immunoassay according to claim 5, wherein said silicon compound is a hydrophobic silane represented by Formula [I] wherein X and Y independently represent hydrogen, C₁-C₆ alkyl, or C₁-C₆ alkoxyl; R³ represents C₂-C₃₀ alkyl, C₂-C₃₀ alkenyl, C₂-C₃₀ alkoxyl, phenyl or substituted phenyl; R⁴ represents C₁-C₃₀ alkyl or C₁-C₃₀ alkoxyl.

7. The carrier for immunoassay according to claim 6, wherein said hydrophobic silane is alkyltrialkoxysilane, vinyltrialkoxysilane or phenyltrialkoxysilane.

8. The carrier for immunoassay according to claim 7, wherein said alkyltrialkoxysilane is octadecyltriethoxysilane.

9. The carrier for immunoassay according to any one of claims 1 to 8, wherein said carrier comprises a substrate coated with said silicon compound.

10. The carrier for immunoassay according to claim 9, wherein said substrate is glass, quartz or ceramics.

11. The carrier for immunoassay according to claim 10, wherein said substrate is glass fiber.

12. The carrier for immunoassay according to any one of claims 9 to 11, wherein said substrate is porous and said surface of said carrier is porous.

13. The carrier for immunoassay according to claim 12, wherein said substrate is in the form of membrane made of glass fibers.

14. A solid phase for immunoassay obtainable by binding an immunoreactive substance to a carrier for immunoassay which comprises, at least on surface thereof, a hydrophobic material, and blocking the resulting carrier by coating exposed area of said hydrophobic material with an amphipathic substance.

15. The solid phase for immunoassay according to claim 14, wherein said carrier for immunoassay is the carrier for immunoassay according to any one of claims 1 to 13.

16. The solid phase for immunoassay according to claim 14 or 15, wherein said amphipathic substance is a surfactant.

17. The solid phase for immunoassay according to claim 16, wherein said surfactant is a nonionic surfactant.
